# EUROPEAN PATENT APPLICATION

(11) **EP 4 595 869 A1**
(43) Date of publication of application: **06.08.2025**
(21) Application number: 23871956.1
(22) Date of filing: 14.09.2023
(51) Int. Cl.: A61B 3/028, A61B 3/036

(54) **SUBJECTIVE OPTOMETRY DEVICE AND SUBJECTIVE OPTOMETRY PROGRAM**

(30) Priority: 30.09.2022 JP 2022157417; 09.06.2023 JP 2023095849
(71) Applicant: NIDEK CO., LTD., Gamagori-shi, Aichi 443-0038 (JP)
(72) Inventor: HORINO, Taeko, Gamagori-shi Aichi 443-0038 (JP); SHIMAZAKI, Arisa, Gamagori-shi Aichi 443-0038 (JP)
(74) Representative: Hoefer & Partner Patentanwälte mbB
(86) International application number: PCT/JP2023/033513
(87) International publication number: WO 2024/070723

(57) **Abstract**

Provided is a subjective optometry device comprising: an optotype presentation means that presents a test optotype to an eye to be examined; a correction means that changes an optical characteristic of an optotype luminous flux emitted from the optotype presentation means; and a control means that, on the basis of a response entered by a test subject, controls the optotype presentation means and/or the correction means, and automatically advances an optometry exam including a plurality of test items. The subjective optometry device further comprises: a first acquisition means that acquires at least any piece of data selected from previous eyeglass data of eyeglasses worn by the test subject, objective measurement data of the eye to be examined, and previous subjective measurement data acquired in the past; a second acquisition means that acquires a correction value by which the eye being examined is corrected by the correction means when the optometry exam advances; a comparison processing means that compares a tolerance value that is based on the data acquired by the first acquisition means with the correction value acquired by the second acquisition means; and an output means that outputs an alert that is based on the comparison result from the comparison means.

## Description

### TECHNICAL FIELD

The present disclosure relates to a subjective optometry device and a subjective optometry program for subjectively measuring an optical characteristic of a subject eye.

### BACKGROUND ART

In the subjective optometry device, a subjective optometry device is used for subjectively measuring optical characteristics of a subject eye. For example, the subjective optometry device can measure the optical characteristics of the subject eye by placing an optical member in front of the subject eye and presenting a visual target to the subject eye via the optical member. For example, a subject uses the subjective optometry device to perform self-optometry in which a subjective examination is progressed by himself or herself (see Patent Literature 1).

### CITATION LIST

### PATENT LITERATURE

Patent Literature 1: JP2022-89255A

### SUMMARY OF INVENTION

However, in the self-optometry using such a subjective optometry device, there are more and more occasions when an examiner is not near the subject. This makes it difficult to check whether the optometry is being performed correctly. Therefore, after the examination is completed, the examiner needs to check examination results and determine whether the optometry is performed correctly. Further, depending on the examination results of the subject eye, it may be necessary to perform the examination again from the beginning.

In view of the above problems, a technical object of the present disclosure is to provide a subjective optometry device and a subjective optometry program that allows an optometry to progress smoothly.

A subjective optometry device according to a first aspect of the present disclosure is for subjectively measuring an optical characteristic of a subject eye, the subjective optometry device including: a visual target presenting unit configured to present an examination visual target to a subject eye; a correction unit configured to change an optical characteristic of a visual target light flux emitted from the visual target presenting unit; a controller configured to control at least one of the visual target presenting unit and the correction unit, based on an answer input by a subject, to automatically progress an optometry including a plurality of examination items; a first acquisition unit configured to acquire at least one of previous eyeglass data obtained by measuring an optical characteristic of eyeglasses worn by a subject, objective measurement data obtained by objectively measuring an optical characteristic of the subject eye, and previous subjective measurement data acquired by subjectively measuring an optical characteristic of the subject eye previously; a second acquisition unit configured to acquire a correction value by which the subject eye is corrected by the correction unit during the optometry; a comparison processing unit configured to compare an allowable value with the correction value that is acquired by the second acquisition unit, the allowable value being based on at least one of the previous eyeglass data, the objective measurement data, and the previous subjective measurement data that are acquired by the first acquisition unit; and an output unit configured to output an alert based on a comparison result of the comparison processing unit.

A subjective optometry program according to a second aspect of the present disclosure is to be used in a subjective optometry device for subjectively measuring an optical characteristic of a subject eye, the subjective optometry device including: a visual target presenting unit configured to present an examination visual target to a subject eye; a correction unit configured to change an optical characteristic of a visual target light flux emitted from the visual target presenting unit; and a controller configured to control at least one of the visual target presenting unit and the correction unit, based on an answer input by a subject, to automatically progress an optometry including a plurality of examination items. The subjective optometry program including instructions which, when executed by a processor of the subjective optometry device, cause the processor to execute: a first acquisition step of acquiring at least one of previous eyeglass data obtained by measuring an optical characteristic of eyeglasses worn by a subject, objective measurement data obtained by objectively measuring an optical characteristic of the subject eye, and previous subjective measurement data acquired by subjectively measuring an optical characteristic of the subject eye previously; a second acquisition step of acquiring a correction value by which the subject eye is corrected by the correction unit during the optometry; a comparison processing step of comparing an allowable value with the correction value that is acquired in the second acquisition step, the allowable value being based on at least one of the previous eyeglass data, the objective measurement data, and the previous subjective measurement data that are acquired in the first acquisition step; and an output step of outputting an alert based on a comparison result of the comparison processing step.

### BRIEF DESCRIPTION OF DRAWINGS

[FIG. 1A] FIG. 1A is an external view of an eye refractive power measurement unit supported at a standby position.
[FIG. 1B] FIG. 1B is an external view of the eye refractive power measurement unit supported at a measurement position.
[FIG. 2A] FIG. 2A is a schematic diagram illustrating an optical arrangement of a light projection optical system during a distance vision examination.
[FIG. 2B] FIG. 2B is a schematic diagram illustrating an optical arrangement of the light projection optical system during a near vision examination.
[FIG. 3] FIG. 3 is a schematic diagram of the eye refractive power measurement unit.
[FIG. 4] FIG. 4 is a schematic diagram of a control system of a subjective optometry device.
[FIG. 5] FIG. 5 is an example of a self-optometry assistance screen.
[FIG. 6] FIG. 6 is an example of the self-optometry assistance screen.
[FIG. 7] FIG. 7 is a diagram illustrating a correspondence relationship between an amount of cylindrical correction and an allowable value for an amount of astigmatic axis correction.

### DESCRIPTION OF EMBODIMENTS

### <Overview>

An embodiment of a subjective optometry device according to the present embodiment will be described. Note that items classified in < > below may be used independently or in conjunction with each other.

The subjective optometry device according to the present embodiment is a device for subjectively measuring an optical characteristic of a subject eye. For example, the optical characteristic of a subject eye may be eye refractive power (as an example, at least one of spherical refractive power, cylindrical refractive power, astigmatic axis angle, and the like), a binocular vision function (as an example, at least one of a prism amount, a stereoscopic vision function, and the like), contrast sensitivity, and the like.

### <Visual Target Presenting Unit>

The subjective optometry device according to the present embodiment may include a visual target presenting unit. The visual target presenting unit is configured to present an examination visual target to a subject eye. For example, the visual target presenting unit may be configured to present the examination visual target to the subject eye by emitting a visual target light flux toward the subject eye.

For example, the visual target presenting unit may be a display (for example, a display 31). For example, the visual target presenting unit may be a light source and a digital micromirror device (DMD). For example, the visual target presenting unit may be a light source and a visual target plate.

For example, the visual target light flux from the visual target presenting unit may be directly guided toward the subject eye. Further, for example, the visual target light flux from the visual target presenting unit may be guided toward the subject eye via a light projection optical system (for example, a light projection optical system 30). For example, the light projection optical system may include at least one optical member for passing the visual target light flux emitted from the visual target presenting unit. As an example, at least one of a lens, a mirror, and the like may be provided.

### <Correction Unit>

The subjective optometry device according to the present embodiment may include a correction unit. The correction unit is configured to change an optical characteristic of the visual target light flux emitted from the visual target presenting unit. For example, the correction unit may be disposed in an optical path of the light projection optical system to change the optical characteristics of the visual target light flux.

For example, the correction unit may have any configuration as long as the optical characteristics of the visual target light flux can be changed.

For example, the correction unit may include an optical element. For example, the optical element may be at least one of a spherical lens, a cylindrical lens, a variable focus lens, a cross cylinder lens, a rotary prism, a wavefront modulating element, and the like. Of course, the optical element may be different from these. In this case, the optical characteristic of the visual target light flux are changed by controlling the optical element.

Further, for example, the correction unit may have a configuration for optically changing a presenting position (presenting distance) of the visual target with respect to the subject eye. As an example, the correction unit may have a configuration configured to move the visual target presenting unit in an optical axis direction, or may have a configuration configured to move an optical element (for example, a spherical lens) in the optical path in the optical axis direction. In this case, the optical characteristic of the visual target light flux is changed by controlling a drive unit for controlling at least one of the visual target presenting unit and the optical element.

Further, for example, the correction unit may be an eye refractive power measurement unit (for example, an eye refractive power measurement unit 40) configured to switch and position an optical element (for example, an optical element 52) via an examination window (for example, an examination window 43) in front of the subject eye. For example, the eye refractive power measurement unit may include a lens disk (for example, a lens disk 50) in which a plurality of optical elements are arranged on the same circumference. In this case, the optical characteristic of the visual target light flux is changed by controlling a drive unit (for example, a drive unit 51, a drive unit 53, and the like) for controlling the lens disk.

### <Control Unit>

The subjective optometry device according to the present embodiment may include a control unit (for example, a controller 60). The control unit is configured to control at least one of the visual target presenting unit and the correction unit based on an answer input by a subject to automatically progresses an optometry including a plurality of examination items. For example, the control unit may be configured to automatically progress the optometry including a plurality of examination items by executing a self-optometry program, based on an operation signal input by an operation on an answer input unit by the subject.

For example, the answer input unit may be unit for the subject to input an answer by himself or herself. As an example, the answer input unit may be an operation unit (for example, a subject controller 20) such as a lever switch or a push button switch.

For example, the self-optometry program may include a self-optometry application program. The self-optometry application program is a program for achieving an application (self-optometry application) that automatically progresses the optometry, based on an answer input by the subject.

For example, the control unit may be configured to control the visual target presenting unit. As an example, the control unit may control the display of the visual target presenting unit and change at least one of the type of the visual target, a visual acuity value of the visual target, and the like. For example, the control unit may be configured to control the correction unit. As an example, the control unit may change at least one of movement of the visual target presenting unit or the optical element in the correction unit, arrangement of the optical element, and the like. Of course, for example, the control unit may combine such control in the visual target presenting unit and the correction unit.

For example, the control unit may be configured to control execution of a plurality of examination items in the optometry of the self-optometry program, based on a comparison result of a comparison processing unit to be described later. For example, the control unit may be configured to omit a predetermined examination item among the plurality of examination items in the optometry, based on the comparison result of the comparison processing unit to progress to the next examination item. In other words, for example, based on the comparison result of the comparison processing unit, the control unit may interrupt the execution of a predetermined examination item being examined on the subject eye, and automatically transition to the next examination item by skipping the acquisition of the examination result in the predetermined examination item and executing the next examination item. For example, in this case, it is possible to reduce the possibility that a reliable examination result cannot be obtained in a predetermined examination item of the subject eye and the examination continues for a long time, so that the optometry can be smoothly progressed to the end.

Further, for example, the control unit may be configured not to perform a predetermined examination item among the plurality of examination items in the optometry, based on the comparison result of the comparison processing unit, to forcibly terminate the optometry. In other words, for example, based on the comparison result of the comparison processing unit, the control unit may terminate the execution of a predetermined examination item being examined on the subject eye and the execution of an examination item scheduled after the predetermined examination item, and may automatically end the optometry. For example, when an examination result with low reliability is obtained for a predetermined examination item of the subject eye, the examination result may affect the examination result of the next examination item. Therefore, for example, it is possible to maintain a certain accuracy by not performing a predetermined examination item and those subsequent to the predetermined examination item.

For example, for examination items for acquiring at least one of the spherical power (amount of spherical correction), the cylindrical power (amount of cylindrical correction), and the astigmatic axis angle (amount of astigmatic axis correction) for correcting the subject eye, the control unit may omit the execution of the examination items or cancel the execution of the examination items based on the comparison result of the comparison processing unit. Further, for example, for an examination item for acquiring the visual acuity value of the subject eye, the control unit may omit the execution of the examination item or cancel the execution of the examination item based on the comparison result of the comparison processing unit.

The control unit according to the present embodiment may omit the execution of the examination item or cancel the execution of the examination item for an examination item for acquiring the astigmatic axis angle as a correction value of the subject eye. For example, in the examination for acquiring the astigmatic axis angle, a subject is required to compare appearances of two examination visual targets and select one of the examination visual targets. For example, in a case where the cylindrical power of the subject eye is weak, the difference in the appearance of the examination visual targets is particularly small, increasing the chances of confusion in the selection. Therefore, it is difficult to obtain a highly reliable examination result, and the examination result may not be determined indefinitely. Therefore, the self-optometry can be smoothly performed by omitting the examination item for acquiring the astigmatic axis angle or by forcibly terminating the subsequent examination item. It is particularly useful to execute such control when the self-optometry includes an examination item for acquiring an astigmatic axis angle among a plurality of examination items.

For example, in a case where a predetermined examination item in the optometry of the self-optometry program is omitted or the optometry is forcibly terminated, the control unit may acquire at least one of previous eyeglass data described later, objective measurement data described later, and previous subjective measurement data described later as an examination result of the predetermined examination item. For example, when the predetermined examination item is omitted, an examination result with high reliability cannot be acquired for the predetermined examination item. Further, for example, in a case where the optometry is forcibly terminated, a highly reliable examination result cannot be acquired for the predetermined examination item, and since the examination is not performed after the predetermined examination item, there is no examination result. Therefore, for example, by acquiring at least one of the previous eyeglass data, the objective measurement data, and the previous subjective measurement data of the subject eye as reference data of the examination result in the predetermined examination item, the examiner can easily grasp the optical characteristics of the subject eye.

### <First Acquisition Unit>

The subjective optometry device according to the present embodiment may include a first acquisition unit (for example, the controller 60). The first acquisition unit is configured to acquire at least one of previous eyeglass data obtained by measuring optical characteristics of glasses worn by the subject, objective measurement data obtained by objectively measuring optical characteristics of the subject eye, and previous subjective measurement data obtained by subjectively measuring optical characteristics of the subject eye in the past. For example, the optical characteristics of the glasses in the previous eyeglass data may include refractive power data of the eyeglass lens (as an example, at least one of spherical data indicating a spherical refractive power, cylindrical data indicating a cylindrical refractive power, astigmatic axis data indicating an astigmatic axis angle, and the like). Of course, in addition to the refractive power data of the eyeglass lens, polarization characteristic data, a pupillary distance, and the like may be included. In addition, for example, the optical characteristics of the subject eye in the objective measurement data may include eye refractive power data of the subject eye (as an example, at least one of spherical data indicating a spherical refractive power, cylindrical data indicating a cylindrical refractive power, astigmatic axis data indicating an astigmatic axis angle, and the like). Of course, in addition to the eye refractive power data of the subject eye, polarization characteristic data, a pupillary distance, and the like may be included. In addition, for example, the optical characteristics of the subject eye in the previous subjective measurement data may include eye refractive power data of the subject eye (as an example, at least one of spherical data indicating a spherical refractive power, cylindrical data indicating a cylindrical refractive power, astigmatic axis data indicating an astigmatic axis angle, and the like). Of course, in addition to the refractive power data of the subject eye, polarization characteristic data, a pupillary distance, and the like may be included.

In the present embodiment, the previous subjective measurement data acquired in the past may be subjective measurement data acquired at a timing before the current time point. For example, the subjective measurement data may be acquired in an examination item executed before a predetermined examination item currently being executed among a plurality of examination items for examining the subject eye. In addition, for example, the subjective measurement data may be acquired at a different date and time using the subjective optometry device according to the present embodiment or a device other than the subjective optometry device according to the present embodiment.

The first acquisition unit of the present embodiment may include astigmatic axis data (astigmatic axis angle) as at least one of the previous eyeglass data, the objective measurement data, and the previous subjective measurement data. That is, the first acquisition unit may be configured to acquire at least one of the astigmatic axis data (astigmatic axis angle) in the previous eyeglass data and the astigmatic axis data (astigmatic axis angle) in the objective measurement data.

Further, the first acquisition unit of the present embodiment may include cylindrical data (cylindrical refractive power) as at least one of the previous eyeglass data, the objective measurement data, and the previous subjective measurement data. That is, the first acquisition unit may be configured to acquire at least one of the cylindrical data (cylindrical refractive power) in the previous eyeglass data and the cylindrical data (cylindrical refractive power) in the objective measurement data.

For example, the first acquisition unit may be configured to acquire at least one of the previous eyeglass data, the objective measurement data, and the previous subjective measurement data input by the operation on an operation unit (for example, an examiner controller 10) by the examiner. In addition, for example, the first acquisition unit may read an identifier of each subject, and acquire at least one of the previous eyeglass data, the objective measurement data, and the previous subjective measurement data stored in the identifier. As an example, an ID, a character string, a one-dimensional code, a two-dimensional code, a color code, or the like may be used as the identifier. In addition, for example, the first acquisition unit may acquire at least one of the previous eyeglass data, the objective measurement data, and the previous subjective measurement data by receiving data measured using a device other than the subjective optometry device according to the present embodiment. As an example, the data may be received from a lens meter, an objective measurement device, and the like.

### <Second Acquisition Unit>

The subjective optometry device according to the present embodiment may include a second acquisition unit (for example, the controller 60). For example, the second acquisition unit is configured to acquire a correction value by which the subject eye is corrected by the correction unit during the progress of the optometry on the subject eye. That is, for example, the second acquisition unit is configured to acquire the correction value that changes with the progress of the optometry on the subject eye. For example, the correction value of the subject eye may be at least one of the spherical power (amount of spherical correction), the cylindrical power (amount of cylindrical correction), and the astigmatic axis angle (amount of astigmatic axis correction) for correcting the subject eye.

The second acquisition unit of the present embodiment may be configured to acquire at least the astigmatic axis angle (amount of astigmatic axis correction) as the correction value for correcting the subject eye.

For example, the second acquisition unit may be configured to acquire the correction value of the subject eye at at least any timing from the start to the end of the optometry including a plurality of examination items for the subject eye. For example, the second acquisition unit may acquire the correction value once from the start to the end of the optometry. For example, the second acquisition unit may acquire the correction value a plurality of times from the start to the end of the optometry. For example, a timing at which the optometry ends may be a timing at which a final examination result of the subject eye is output.

For example, the second acquisition unit may be configured to acquire an amount of correction at a predetermined timing during the examination of a predetermined examination item in a plurality of examination items for the subject eye. For example, the predetermined timing may be at least one of a timing at which a certain time has elapsed, a timing at which a predetermined control operation is executed, and the like. For example, the second acquisition unit may be configured to acquire the correction value once during the examination of the predetermined examination item for the subject eye. Further, for example, the second acquisition unit may be configured to acquire the correction value a plurality of times during the examination of the predetermined examination item for the subject eye. In this case, the second acquisition unit may be configured to acquire the correction value of the subject eye every time an operation signal from the answer input unit by the subject is received during the examination of the predetermined examination item for the subject eye. In this case, the second acquisition unit may be configured to acquire the correction value of the subject eye every time at least one of the visual target presenting unit and the correction unit is controlled during the examination of the predetermined examination item for the subject eye.

In the present embodiment, the predetermined timing may be a timing each time the control unit controls the correction unit to change the optical characteristic of the visual target light flux. That is, the correction value of the subject eye may be sequentially acquired every time the correction value of the subject eye is changed.

### <Comparison Processing Unit>

The subjective optometry device according to the present embodiment may include a comparison processing unit (for example, the controller 60). The comparison processing unit is configured to compare an allowable value based on at least one of the previous eyeglass data, the objective measurement data, and the previous subjective measurement data which are acquired by the first acquisition unit, with the correction value acquired by the second acquisition unit. For example, the comparison processing unit may be configured to compare an allowable value based on at least one of the previous eyeglass data, the objective measurement data, and the previous subjective measurement data acquired by the first acquisition unit, with the correction value acquired by the second acquisition unit at a predetermined timing.

For example, the comparison processing unit may be configured to compare an allowable value based on at least one of the previous eyeglass data, the objective measurement data, and the previous subjective measurement data which are acquired by the first acquisition unit with the correction value acquired by the second acquisition unit. For example, the comparison processing unit may be configured to directly compare the allowable value with the correction value. For example, the comparison processing unit may be configured to execute a difference process as a comparison process between the allowable value and the correction value. Thereby, for example, a difference result of optical characteristics (for example, eye refractive power) between the allowable value and the correction value may be acquired. In addition, for example, the comparison processing unit may indirectly compare the allowable value with the correction value by executing a comparison process between the correction value and deviation information indicating a deviation between the allowable value and the correction value. For example, the deviation information based on the allowable value and the correction value may be obtained by such a difference process.

For example, the comparison processing unit may be configured to compare an allowable value based on the astigmatic axis data included in at least one of the previous eyeglass data, the objective measurement data, and the previous subjective measurement data acquired by the first acquisition unit with an astigmatic axis angle included in the correction value acquired by the second acquisition unit. For example, in the examination of the astigmatic axis angle, the subject is required to select one of the examination visual targets having a different appearance. However, in a case where the selection is difficult, the correction value is likely to greatly deviate from the previous eyeglass data, the objective measurement data, or the previous subjective measurement data. Therefore, by comparing the allowable value based on the astigmatic axis data with the astigmatic axis angle in the correction value, in particular, it is possible to easily check whether the examination of the astigmatic axis angle, which is more likely to deviate from the correction value than other examinations, is performed properly.

For example, the allowable value may be a value for detecting whether or not to output an alert. For example, the allowable value may be any value that can be set by the examiner, or may be a fixed value set in advance by experiment or simulation. For example, the allowable value may be set as a predetermined threshold. As an example, the predetermined threshold may be the same value as the previous eyeglass data, the objective measurement data, or the previous subjective measurement data. As an example, the predetermined threshold may be a value different from the previous eyeglass data, the objective measurement data, or the previous subjective measurement data. Such a predetermined threshold value may be one or more. For example, when a plurality of predetermined thresholds are provided as the allowable values, the allowable values may be provided as the predetermined allowable range.

For example, the allowable value may be provided as a uniform value regardless of the optical characteristics of the previous eyeglass data, the objective measurement data, or the previous subjective measurement data acquired by the first acquisition unit. In addition, for example, the allowable value may be set for each optical characteristic of the previous eyeglass data, the objective measurement data, or the previous subjective measurement data acquired by the first acquisition unit. For example, in this case, the allowable value may be changed according to whether or not the optical characteristic of the previous eyeglass data, the objective measurement data, or the previous subjective measurement data exceeds a predetermined optical characteristic (as an example, eye refractive power). As an example, the allowable value may be changed between less than 5.0 D and 5.0 D or more. In addition, for example, in this case, the allowable value may be changed according to the stage of the optical characteristic (eye refractive power) of the previous eyeglass data, the objective measurement data, or the previous subjective measurement data. As an example, the allowable value may be changed each time the optical characteristic of the previous eyeglass data, the objective measurement data, or the previous subjective measurement data changes by 0.25 D units, 1.0 D units, 5.0 D units, or the like. For example, depending on the magnitude of the optical characteristics of the previous eyeglass data, the objective measurement data, or the previous subjective measurement data, the susceptibility of the examination result to deviations with respect to the optical characteristics varies. Therefore, by setting different allowable values for the optical characteristics of the previous eyeglass data, the objective measurement data, or the previous subjective measurement data, an alert is appropriately output by the output unit described later.

For example, the allowable value may be set more strictly as the cylindrical data in at least one of the previous eyeglass data, the objective measurement data, and the previous subjective measurement data has a higher refractive power. For example, when first cylindrical data has a high refractive power (for example, -3.00 D) and second cylindrical data has a lower refractive power (for example, - 0.50 D) than the first cylindrical data, the first cylindrical data may be set to have a smaller allowable value than the second cylindrical data. As an example, when the allowable value is provided as a predetermined threshold value, an allowable value (threshold value) of the first cylindrical data may be set to be smaller than an allowable value (threshold value) of the second cylindrical data. Further, as an example, when the allowable value is provided as a predetermined allowable range, an allowable value (allowable range) of the first cylindrical data may be set to be narrower than an allowable value (allowable range) of the second cylindrical data.

For example, the higher the refractive power of the cylindrical data of the subject eye, the more likely it is that a difference in the appearances of the examination visual targets appears in an astigmatic axis examination. Therefore, an allowable range of axis deviation in the astigmatic axis angle in a subjective measurement may be set to be narrower with respect to the astigmatic axis angle of the previous eyeglass data, the objective measurement data, or the previous subjective measurement data. On the other hand, for example, the lower the refractive power of the cylindrical data of the subject eye, the less likely it is that a difference in the appearances of the examination visual targets appears in an astigmatic axis examination. Therefore, an allowable range of axis deviation in the astigmatic axis angle in a subjective measurement may be set to be wider with respect to the astigmatic axis angle of the previous eyeglass data, the objective measurement data, or the previous subjective measurement data. Therefore, by setting the allowable value according to the cylindrical data of the previous eyeglass data, the objective measurement data, or the previous subjective measurement data, the alert is appropriately output by the output unit described later.

### <Output Unit>

The subjective optometry device according to the present embodiment includes an output unit (for example, the controller 60). The output unit is configured to output an alert based on a comparison result of the comparison processing unit. For example, the alert may inform an abnormality in the examination of the subject eye. For example, the alert may enable identification of whether the examination of the subject eye is normal or abnormal. For example, the alert may identify that the examination of the subject eye is normal. For example, the alert may identify that the examination of the subject eye is abnormal.

As an example, the alert may be output in a format different from the output when the self-optometry is normally executed. In this case, the examination result for each examination item may be output in at least one of blank fields, filling, color coding, and the like. As an example, the alert may be an alert for notifying that the correction value of the subject eye exceeds an allowable value based on at least one of the previous eyeglass data, the objective measurement data, and the previous subjective measurement data. In other words, the alert may be an alert for supporting the determination of whether the correction value of the subject eye is appropriate.

For example, the alert may be output to the examiner to notify the examiner of an abnormality in the examination. For example, the alert may be output to the subject to notify the examiner of an abnormality in the examination. As an example, in this case, an alert for guiding the subject to call the examiner may be output. As a result, since the examiner is called by the subject, the examiner can be notified of the abnormality in the examination. The alert may be output to at least one of the examiner and the subject. For example, when notifying the examiner of an abnormality in the examination, it may be possible to notify the examiner only to prevent the subject from becoming worried.

For example, the alert may be configured to notify that there is an abnormality in the examination. For example, the alert may be configured to indicate a message on a display unit. Further, for example, the alert may be configured to emphasize a screen on the display unit. As an example, at least one of inversion or change of the color of the screen, flashing of a screen, change of a display size, and the like may be used. For example, the alert may be configured to indicate a sign (for example, at least one of a window, a mark, an icon, a character, a number, and a symbol) on the display unit. Of course, for example, the alert may be configured to emphasize such a sign.

For example, the output unit may be configured to control the display unit to display the alert on the display unit. Further, for example, the output unit may be configured to control a sound generation unit (as an example, a speaker) to cause the sound generation unit to generate an alert as a sound. Further, for example, the output unit may be configured to control a notification unit (as an example, a lamp) to indicate the alert by lighting or blinking the notification unit. Further, for example, the output unit may be configured to control a printing unit (as an example, a printer) to cause the printing unit to print an alert. Further, for example, the output unit may be configured to control an external storage unit (as an example, a memory or a server) and transmit an alert to the external storage unit. Of course, for example, the output unit may be configured to execute a combination of these controls or may execute a control different from these controls. Accordingly, the examiner can easily check the examination status, and can smoothly perform the examination.

For example, the output unit may be configured to output an alert during examination of a predetermined examination item among a plurality of examination items for the subject eye. For example, the output unit may be configured to output the alert at any timing during the examination of the predetermined examination item. As an example, in the examination of the predetermined examination item, the alert may be output at at least one of a timing at which a certain time has elapsed, a timing at which the correction value of the subject eye changes, a timing at which the correction value of the subject eye is determined, and the like. For example, by outputting the alert during the examination of the predetermined examination item in this way, it is possible to easily determine whether the correction value of the subject eye exceeds the allowable value. Further, by outputting the alert during the examination of the predetermined examination item, it is possible to easily determine the next operation to take, such as whether to continue or interrupt the examination of the predetermined examination item.

For example, the output unit may output an alert when a plurality of examination items for the subject eye are completed. For example, by outputting the alert at a timing when the plurality of examination items are completed, it is possible to easily determine whether the correction value of the subject eye exceeds the allowable value. Therefore, the examiner can easily determine whether there is any problem in the examination result.

For example, the output unit may be configured to execute at least one of the output of an alert during the examination of the predetermined examination item as described above and the output of an alert when the plurality of examination items are completed. Of course, both the output of the alert during the examination of the predetermined examination item and the output of the alert when the plurality of examination items are completed may be executed.

The subjective optometry device according to the present embodiment may execute at least one of control for progressing a next examination item by omitting a predetermined examination item in the optometry and output of an alert based on a comparison result of the comparison processing unit. In addition, the subjective optometry device according to the present embodiment may execute at least one of control for forcibly terminating the optometry without executing a predetermined examination item in the optometry and output of an alert based on a comparison result of the comparison processing unit.

The present disclosure is not limited to the device described in the present embodiment. For example, terminal control software (a program) that performs functions of the above-described embodiment may be supplied to a device or a system via a network or various storage media, and a control device (for example, a CPU or the like) of the device or the system may read and execute the program.

### <Example>

An example of the subjective optometry device according to the present embodiment will be described. In the present example, a subjective optometry device for subjectively measuring an optical characteristic of a subject eye, which integrally includes a display configured to display an examination visual target to the subject eye and an eye refractive power measurement unit configured to change an optical characteristic of a visual target light flux emitted from the display, will be described. Of course, for example, the display may be provided as a housing separate from the subjective optometry device.

### <Appearance of Device>

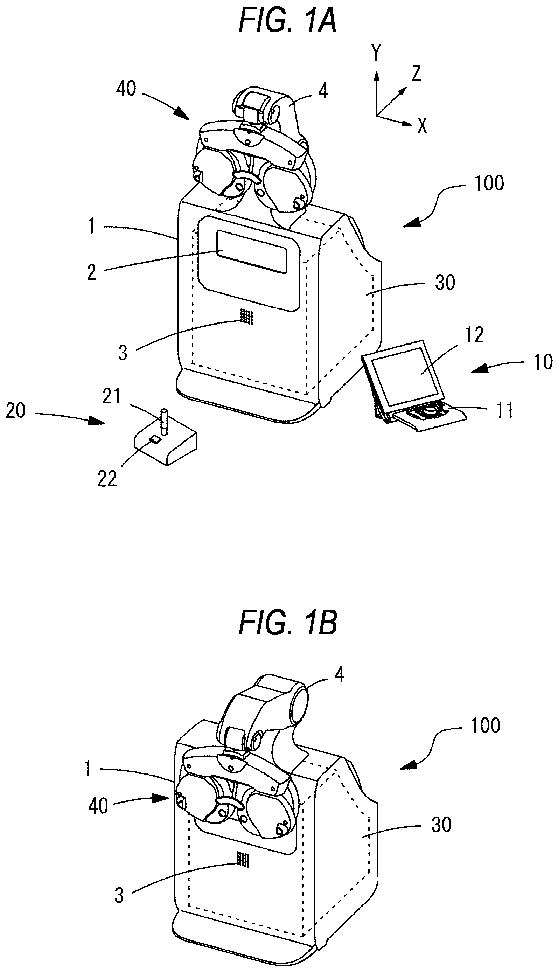

FIGS. 1A and 1B are external views of a subjective optometry device 100. FIG. 1A shows a state in which an eye refractive power measurement unit 40 is supported at a standby position. FIG. 1B shows a state in which the eye refractive power measurement unit 40 is supported at a measurement position. For example, the subjective optometry device 100 includes a housing 1, a presentation window 2, a speaker 3, a holding unit 4, an examiner controller 10, a subject controller 20, the eye refractive power measurement unit 40, and the like.

The housing 1 includes a light projection optical system 30 therein. The presentation window 2 is configured to transmit a visual target light flux by the light projection optical system 30. The visual target light flux is projected onto a subject eye E through the presentation window 2. When the eye refractive power measurement unit 40 is disposed between the subject eye E and the presentation window 2 (see FIG. 1B), the visual target light flux is projected on the subject eye E through the presentation window 2 and the examination window 43 to be described later. Accordingly, an examination visual target is presented on the subject eye E. The speaker 3 is configured to output a voice guide or the like.

The holding unit 4 is configured to hold the eye refractive power measurement unit 40. For example, the holding unit 4 is configured to move an arm by driving a drive unit (such as a motor), which is not shown, to move the eye refractive power measurement unit 40 coupled to the arm. This allows the eye refractive power measurement unit 40 to be switched between the standby position and the measurement position.

The examiner controller 10 is used by an examiner to operate the subjective optometry device 100. The examiner controller 10 includes a switch unit 11, a monitor 12, and the like. The switch unit 11 inputs a signal for performing various settings (for example, movement of the eye refractive power measurement unit 40). The monitor 12 is configured to display a self-optometry assistance screen to be described later. For example, various types of information (for example, an examination result of the subject eye E) may be displayed on the self-optometry assistance screen. The monitor 12 may function as a touch panel that also serves as the switch unit 11. A signal from the examiner controller 10 is output to the controller 60 by wired communication or wireless communication.

The subject controller 20 is used to input an answer of the subject. The subject controller 20 includes an answer lever 21, an answer button 22, and the like. The answer lever 21 is used when the subject inputs a direction of an examination visual target. For example, signals in four directions of up, down, left, and right can be input by a tilting operation. The answer button 22 is used when the subject does not select a direction of the examination visual target. A signal from the subject controller 20 is output to the controller 60 by wired communication or wireless communication.

### <Light Projection Optical System>

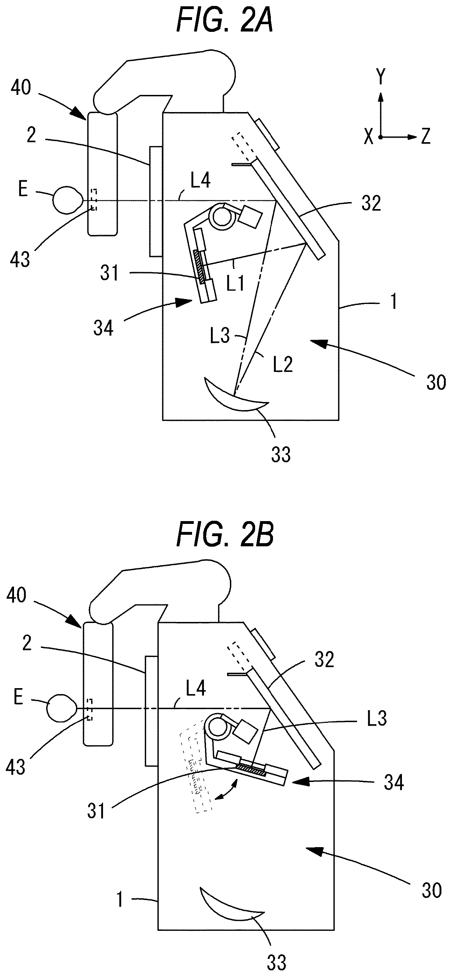

FIGS. 2A and 2B are schematic diagrams of the light projection optical system 30. FIG. 2A shows an optical arrangement at the time of a distance vision examination. FIG. 2B shows an optical arrangement at the time of a near vision examination. The light projection optical system 30 is configured to project a visual target light flux toward the subject eye E. For example, the light projection optical system 30 includes a display 31, a flat mirror 32, a concave mirror 33, and a distance-near switching unit 34.

The display 31 is configured to display a visual target (for example, a fixation target, an examination visual target, or the like). The visual target light flux emitted from the display 31 forms an image on the fundus of the subject eye E, so that a visual target is presented to the subject eye E. For example, the display 31 may be a liquid crystal display (LCD), an organic electro luminescence (EL), a plasma display, or the like.

The display 31 may include a polarizing optical member. For example, the polarizing optical member may be disposed on a front surface of the display 31, or the polarizing optical member may be incorporated and integrally disposed in the display 31. Thus, the display 31 can emit linearly polarized light or circularly polarized light that is polarized in a predetermined direction (vertical, horizontal, 45-degree diagonal, or the like).

The flat mirror 32 is configured to reflect the visual target light flux from the display 31 and guide the visual target light flux to the concave mirror 33. The flat mirror 32 is configured to reflect the visual target light flux from the display 31 and guides the visual target light flux to the subject eye E. For example, the flat mirror 32 is disposed such that a distance (presenting distance) from the subject eye E to the display 31 is optically 40 cm at the time of the near vision examination of the subject eye E. Instead of the flat mirror 32, a reflection member such as a prism, a beam splitter, or a half mirror may be used.

The concave mirror 33 reflects the visual target light flux from the display 31 and guides the visual target light flux to the flat mirror 32. For example, the concave mirror 33 is disposed such that a distance (presenting distance) from the subject eye E to the display 31 is optically 5 m at the time of the distance vision examination of the subject eye E. Instead of the concave mirror 33, a reflection member such as an aspherical mirror or a free curved surface mirror may be used. A lens or the like may be used instead of the concave mirror 33.

The distance-near switching unit 34 switches the arrangement of the display 31 between the distance vision examination and the near vision examination of the subject eye E. For example, the distance-near switching unit 34 moves the holding unit by being driven by a drive unit (such as a motor) (not shown), thereby moving the display 31 held by the holding unit. This allows the display 31 to be switched between the distance vision arrangement and the near vision arrangement.

For example, at the time of the distance vision examination for the subject eye E, a display screen of the display 31 is directed to a back surface of the housing 1 (see FIG. 2A). The visual target light flux from the display 31 passes through an optical axis L1 and enters the flat mirror 32, and is reflected by the flat mirror 32 in a direction of an optical axis L2. In addition, the visual target light flux passes through the optical axis L2 and enters the concave mirror 33, and is reflected by the concave mirror 33 in a direction of an optical axis L3. Further, the visual target light flux passes through the optical axis L3 and enters the flat mirror 32, and is reflected by the flat mirror 32 in a direction of an optical axis L4. Accordingly, the visual target light flux emitted to the outside of the housing 1 is projected onto the subject eye E passing through each optical member inside the housing 1.

For example, at the time of the near vision examination for the subject eye E, a display screen of the display 31 is directed to an upper surface of the housing 1 (see FIG. 2B). The visual target light flux from the display 31 passes through the optical axis L3 and enters the flat mirror 32, and is reflected by the flat mirror 32 in a direction of the optical axis L4. Accordingly, the visual target light flux emitted to the outside of the housing 1 is projected onto the subject eye E passing through each optical member inside the housing 1.

### <Eye Refractive Power Measurement Unit (Correction Optical System)>

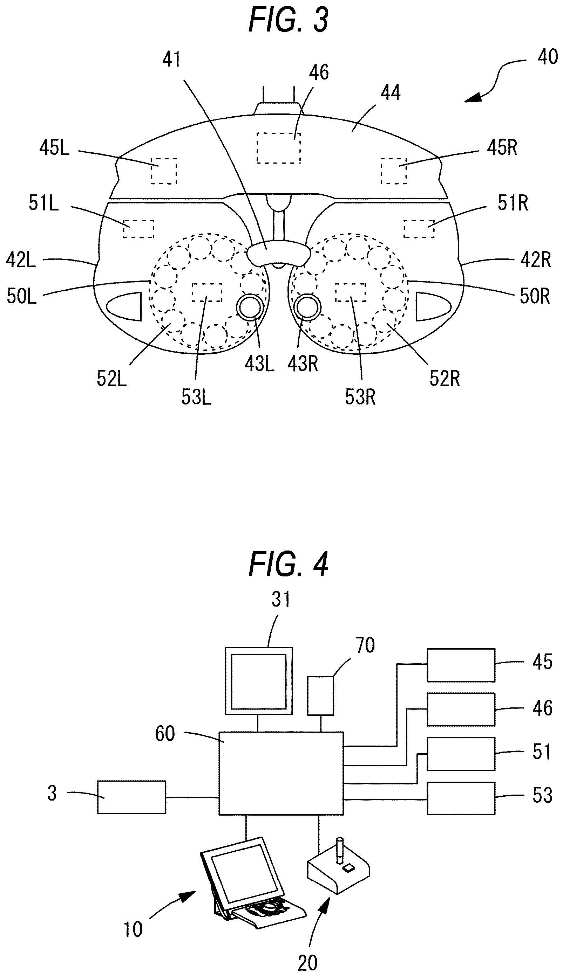

FIG. 3 is a schematic diagram of the eye refractive power measurement unit 40. The eye refractive power measurement unit 40 is configured to subjectively measure a refractive power of the subject eye E. The eye refractive power measurement unit 40 is used as a correction optical system. The correction optical system is arranged in an optical path of the light projection optical system 30 to change an optical characteristic of the visual target light flux. For example, the eye refractive power measurement unit 40 includes a forehead rest 41, a lens unit 42, the examination window 43, a moving unit 44, and the like.

The forehead rest 41 comes into contact with the head of the subject to fix the subject eye E at a predetermined examination position and keep a distance from the subject eye E to the examination window 43 constant. The lens unit 42 includes a left lens unit 42L and a right lens unit 42R, as a pair. The lens unit 42 includes the examination window 43 (a left examination window 43L and a right examination window 43R).

The moving unit 44 is configured to adjust an interval between the left lens unit 42L and the right lens unit 42R and a convergence angle (inward angle) of the left lens unit 42L and the right lens unit 42R. For example, the moving unit 44 adjusts the interval between the left lens unit 42L and the right lens unit 42R by being driven by a drive unit 45 (a left drive unit 45L and a right drive unit 45R). For example, the moving unit 44 adjusts the convergence angle of the left lens unit 42L and the right lens unit 42R by being driven by a drive unit 46. For a detailed configuration of the moving unit 44, for example, refer to JP2004-329345A.

The lens unit 42 includes the lens disk 50 therein. The lens disk 50 includes a left lens disk 50L and a right lens disk 50R, as a pair. The lens disk 50 is rotated by driving of the drive unit 51 (a left drive unit 51L and a right drive unit 51R). In the lens disk 50, an aperture (or a 0 D lens) and a plurality of optical elements 52 (a left optical element 52L and a right optical element 52R) are arranged on the same circumference. These optical elements are rotated by driving of the drive unit 53 (a left drive unit 53L and a right drive unit 53R). Thus, the desired optical element 52 is switched into a position at a desired angle in the examination window 43.

The lens disk 50 includes one lens disk or a plurality of lens disks. For example, a spherical lens disk, a cylindrical lens disk, an auxiliary lens disk, or the like may be provided. As an example, the spherical lens disk may include a plurality of spherical lenses having different spherical powers (spherical refractive powers). As an example, the cylindrical lens disk may include a plurality of cylindrical lenses having different cylindrical powers (cylindrical refractive powers). In addition, as an example, the auxiliary lens disk may include a shielding plate, a polarizing lens (polarizing lenses 54L and 54R), a red filter or a green filter, a dispersion prism, a Maddox lens, a rotary prism, a cross cylinder lens, an autocross cylinder lens, an alignment lens, and the like. The drive unit 51 and the drive unit 53 may be provided for each lens disk.

The eye refractive power measurement unit 40 may be any unit capable of changing the optical characteristics of the visual target light flux. For example, as in the present example, the eye refractive power measurement unit is configured to control the optical element. Further, for example, the eye refractive power measurement unit may be configured to control the wavefront modulating element.

### <Control Unit>

FIG. 4 is a schematic diagram of a control system of the subjective optometry device 100. For example, the controller 60 includes a CPU (a processor), a RAM, a ROM, and the like. For example, the CPU controls each member in the subjective optometry device 100. For example, the RAM temporarily stores various types of information. For example, the ROM stores various programs for controlling the operation of the subjective optometry device 100, examination visual target data, and the like. As an example, a program for achieving an application (self-optometry application) that automatically progresses the optometry based on an answer input by the subject is stored. The controller 60 may be implemented by a plurality of control units (that is, a plurality of processors).

The controller 60 is connected to the speaker 3, the display 31, the examiner controller 10, the subject controller 20, a non-volatile memory 70 (hereinafter, memory 70), and the like. The controller 60 is connected to the drive unit of the holding unit 4, the drive unit of the distance-near switching unit 34, the drive unit of the eye refractive power measurement unit 40 (drive units 45, 46, 51, 53), and the like.

The memory 70 is a non-transitory storage medium capable of storing storage contents even when a supply of power is cut off. For example, a hard disk drive, a flash ROM, a USB memory, or the like can be used as the memory 70.

### <Control Operation>

A control operation in the subjective optometry device 100 having the above-described configuration will be described.

For example, in the self-optometry, subjective examinations are sequentially performed on the subject eye of the subject. For example, based on the objective eye refractive power (objective value) of the subject eye, the subject eye is corrected to a predetermined eye refractive power (0 D or the like) by a predetermined amount of correction, and a subjective examination of the subject eye is performed to obtain a subjective value of the subject eye.

For example, the subjective examination includes a plurality of examination items. As an example, a red-green test may be included to adjust the amount of spherical correction in an initial state of the subject eye E to an appropriate amount of spherical correction. As an example, a cross cylinder test may be included to adjust the amount of cylindrical correction and the amount of astigmatic axis correction in the initial state of the subject eye E to an appropriate amount of cylindrical correction and an appropriate amount of astigmatic axis correction. As an example, a visual acuity test may be included to measure the maximum visual acuity value in a state where the amount of spherical correction, the amount of cylindrical correction, and the amount of astigmatic axis correction of the subject eye E are corrected to an appropriate amount of spherical correction, an appropriate amount of cylindrical correction, and an appropriate amount of astigmatic axis correction. For example, the optometry in each examination item is automatically progressed by executing the self-optometry application.

### <Align Subject Eye>

First, the examiner operates the examiner controller 10 to select a switch (not shown) for lowering the eye refractive power measurement unit 40. For example, the controller 60 drives the drive unit of the holding unit 4 based on the output of an operation signal from the examiner controller 10. For example, the eye refractive power measurement unit 40 is moved to the examination position accordingly (see FIG. 1B), and the eye refractive power measurement unit 40 is disposed on the optical axis L4.

Subsequently, the examiner aligns the subject eye E with the eye refractive power measurement unit 40. For example, the examiner instructs the subject to bring the face into contact with the forehead rest 41 and look into the examination window 43. The subject looks into the examination window 43 in response to an instruction from the examiner. The examiner operates a forehead rest adjustment knob (not shown) to adjust a position of the forehead rest 41 so that a cornea apex distance VD of the subject eye becomes a predetermined distance (for example, 12 mm). Further, for example, the examiner operates the examiner controller 10 to input a pupillary distance PD of the subject eye E measured in advance. For example, the controller 60 drives the drive unit 45 to adjust an interval between the left and right lens units 42 and change an interval between the examination windows 43 according to the pupillary distance PD of the subject eye. Thus, the subject eye E is aligned.

### <Acquire Objective Eye Refractive Power of Subject Eye>

Subsequently, the examiner acquires the objective eye refractive power of the subject eye E. For example, an objective eye refractive power measured in advance using an objective eye refractive power measurement device is acquired. For example, the examiner operates the examiner controller 10 to input a subject ID and operates a predetermined switch. For example, the controller 60 outputs an operation signal to the objective eye refractive power measurement device so as to transmit the objective eye refractive power associated with the subject ID based on the input signal from the switch. For example, when receiving the subject ID from the subjective optometry device 100, the control unit of the objective eye refractive power measurement device calls up the objective eye refractive power corresponding to the subject ID from the memory and transmits the objective eye refractive power to the subjective optometry device 100. The controller 60 of the subjective optometry device 100 receives the objective eye refractive power transmitted from the objective eye refractive power measurement device. Accordingly, the objective eye refractive power of the subject eye E can be acquired. For example, in this example, it is assumed that the objective eye refractive power obtained is 0.00 D in spherical power, -1.00 D in cylindrical power, and 30 degrees in astigmatic axis angle.

### <Acquire Allowable Value>

Subsequently, the controller 60 acquires an allowable value which is based on the acquired objective eye refractive power. For example, in this example, the same allowable value (the same allowable range) is applied regardless of the value of the objective eye refractive power. For example, regarding the objective eye refractive power, the allowable value for spherical power is ±0.50 D regardless of the value. For example, the allowable value for cylindrical power is ±0.50 D regardless of the value. For example, the allowable value for astigmatic axis angle is ±15 degrees regardless of the value.

In this example, since the spherical power in the objective eye refractive power is 0.00 D, the allowable value for the amount of spherical correction of the subject eye is -0.50 D to +0.50 D. In addition, since the cylindrical power in the objective eye refractive power is -1.00 D, the allowable value for the amount of cylindrical correction of the subject eye is -1.50 D to -0.50 D. In addition, since the astigmatic axis angle of the objective eye refractive power is 30 degrees, the allowable value for the amount of astigmatic axis correction of the subject eye is 15 degrees to 45 degrees.

### <Subjective Examination>

For example, the controller 60 sets the objective eye refractive power as an initial value of the subjective examination. For example, the controller 60 controls the eye refractive power measurement unit 40 to place the optical element 52 having a predetermined spherical power and the optical element 52 having a cylindrical power in the examination window 43. Further, for example, the controller 60 places the optical element 52 having a predetermined cylindrical power in the examination window 43 at a predetermined astigmatic axis angle. Accordingly, the subject eye E is corrected to the initial value.

Note that, for the initial value of the subject eye E, it is also possible to use lens information (as an example, optical characteristics of an eyeglass lens) of the previous eyeglasses of the subject measured by an eyeglass lens measurement device. In addition, for the initial value of the subject eye E, it is also possible to use a measurement result of subjective measurement for the subject measured by the subjective optometry device 100 according to the present example or a subjective optometry device different from the subjective optometry device 100 according to the present example.

After correcting the subject eye E with the initial value, the examiner presses a button (not shown) for starting the self-optometry. The controller 60 starts the self-optometry for the subject eye E based on an input signal from a button (not shown) for starting the self-optometry. As an example, in the self-optometry, a red-green test (S), a cross cylinder test (A), a cross cylinder test (C), a red-green test, and a VA (visual acuity) test are sequentially performed on the subject eye E. In the red-green test (S), the spherical power of the subject eye is measured. In the cross cylinder test (A), the astigmatic axis angle of the subject eye is measured. In the cross cylinder test (C), the cylindrical power of the subject eye is measured. In the subsequent red-green test, it is checked whether the adjustment by the subject eye is working in the performed test. In the VA (visual acuity) test, the maximum visual acuity of the subject eye is measured. For example, the subject checks the examination visual target according to the voice guide, and operates the answer lever 21 or the answer button 22 of the subject controller 20. The controller 60 progresses the self-optometry based on the operation signal from the subject controller 20.

Here, in the present example, in each examination on the subject eye, when the amount of correction for correcting the subject eye exceeds an allowable value which is based on the objective eye refractive power, an alert is output. For example, in the red-green test (S), an alert is output when the amount of spherical correction of the subject eye does not fall within a range of -0.50 D to +0.50 D. Similarly, in the cross cylinder test (A), an alert is output when the amount of astigmatic axis correction of the subject eye does not fall within a range of 15 degrees to 45 degrees. Similarly, in the cross cylinder test (C), an alert is output when the amount of cylindrical correction of the subject eye does not fall within a range of -1.50 D to -0.50 D. Hereinafter, the acquisition of the astigmatic axis angle and the output of an alert in the cross cylinder test (A) will be described in detail as an example.

### <Acquire Amount of Astigmatic Axis Correction>

For example, in the cross cylinder test (A) in the example, two point group visual targets are presented to the subject eye E, and it is determined whether the astigmatic axis angle of the subject eye E matches a desired amount of astigmatic axis correction for correcting the subject eye E. For example, if the appearances of the two point group visual targets are seen to the same extent, it is determined that the angles match. For example, if there is a difference between the appearances of the two point group visual targets, it is determined that the angles do not match.

For example, an automatic cross cylinder method is used for the cross cylinder test during the self-optometry. The automatic cross cylinder method is a method in which two point group visual targets are simultaneously presented to the subject by the arrangement of the autocross cylinder lens, and the subject is made to visually recognize and compare the two point group visual targets. Here, a case where the left eye is the measurement eye and the right eye is the non-measurement eye will be described as an example.

For example, the controller 60 drives the left drive unit 51L to arrange the autocross cylinder lens in the left examination window 43L. For example, the autocross cylinder lens is a lens that is divided into two parts with the lens center as an axis, and has two prism regions to which cylindrical powers are given so that the astigmatic axes are orthogonal to each other. Further, the controller 60 drives the right drive unit 51R to dispose a shielding plate in the right examination window 43R. Further, the controller 60 controls the display 31 to display one point group visual target on the screen.

The subject brings the forehead into contact with the forehead rest 41 and observes the point group visual target through the examination window 43 and the presentation window 2. For example, the field of view of the left eye is separated into two by a prism component of the autocross cylinder lens. That is, one point group visual target displayed on the display 31 is presented separately into two point group visual targets via two prism regions. For example, the field of view of the right eye (non-measurement eye) is shielded by the shielding plate.

The controller 60 progresses the cross cylinder test. For example, at the start of the cross cylinder test, the controller 60 causes the speaker 3 to generate a voice guide indicating how to operate the subject controller 20. As an example, a voice guide is generated to indicate that tilt the answer lever 21 to a direction of the clearly visible point group visual target of the two point group visual targets, and press the answer button 22 if both are the same. For example, the controller 60 drives the left drive unit 53L to rotate the autocross cylinder lens of the left examination window 43L to an axial angle for adjusting the astigmatic axis angle. In addition, for example, a voice guide is generated from the speaker 3, asking the subject to indicate a direction of the point group visual target that can be clearly visually recognized.

When the input signal from the answer lever 21 is obtained (when the direction of the point group visual target is selected), the controller 60 rotates the autocross cylinder lens according to a tilting direction of the answer lever 21 to change the astigmatic axis angle. In addition, after changing the astigmatic axis angle, the controller 60 generates a voice guide again, asking the direction of the point group visual target. For example, every time the astigmatic axis angle is changed in this way, the controller 60 acquires a value of the changed astigmatic axis angle as the current amount of astigmatic axis correction.

### <Compare Amount of Astigmatic Axis Correction with Allowable Value>

For example, in the cross cylinder test (A), the subject is required to answer the question by comparing the appearances of the two point group visual targets, and thus the subject may be unsure how to answer the question. In particular, when the difference in appearance between the two point group visual targets is small, there are more opportunities for the subject to be unsure of the answer. For example, if the examination is progressed while the subject continues to give vague answers, a correct examination result for the astigmatic axis angle cannot be obtained, which may affect the progress and examination results of the subsequent examination.

Therefore, in this example, it is detected whether the amount of correction of the subject eye which is sequentially changed during the cross cylinder test exceeds the allowable value which is based on the objective eye refractive power. For example, as described above, the astigmatic axis angle in the objective eye refractive power is 30 degrees, and the allowable value is ±15 degrees. Here, it is assumed that 50 degrees is obtained as the current amount of astigmatic axis correction of the subject eye.

For example, the controller 60 compares the current amount of astigmatic axis correction with the allowable value. For example, the controller 60 compares the current amount of astigmatic axis correction (50 degrees) with the allowable value (15 degrees to 45 degrees). As an example, it is determined whether the current amount of astigmatic axis correction is equal to or less than the upper limit of the allowable value and equal to or more than the lower limit of the allowable value. Thereby, for example, it is detected whether the current amount of astigmatic axis correction falls within the allowable value.

### <Output Alert>

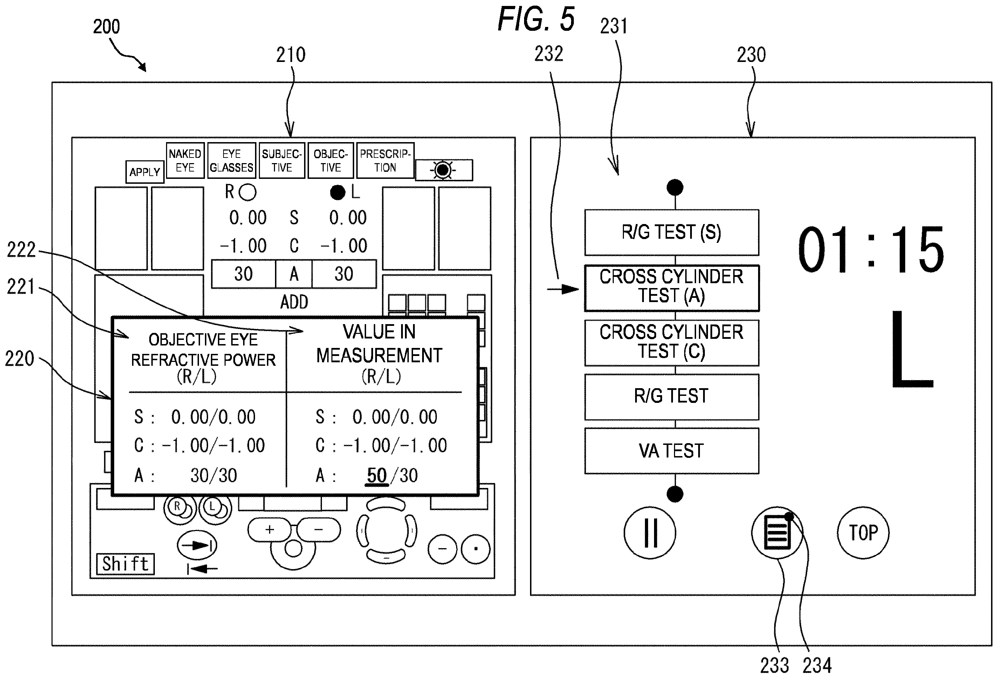

FIG. 5 is an example of a self-optometry assistance screen displayed on the monitor 12 of the examiner controller 10. For example, a self-optometry assistance screen 200 includes an operation image region 210, a parameter screen 220, a progress procedure display region 230, and the like.

The operation image region 210 displays an operation image including information on optical characteristics (eye refractive power) of the subject eye. For example, in the operation image, a value related to the optical characteristic of the subject eye is displayed for each type of optical characteristic. The examiner can specify a value for each type of a plurality of optical characteristics by operating various buttons, values, and the like on the operation image region 210 via a touch panel or the switch unit 11. When the self-optometry is appropriately executed, the correction value of the optical characteristic displayed in the operation image becomes a measurement value of the optical characteristic of the subject eye.

The parameter screen 220 is displayed when a parameter button 233 to be described later is pressed. The parameter screen 220 displays a value 221 of the objective eye refractive power of the subject eye and a current correction value 222 (or an examination result) of the subject eye. As an example, the eye refractive power (spherical refractive power, cylindrical refractive power, astigmatic axis angle, and the like) is displayed in the value 221 of the objective eye refractive power of the subject eye and the current correction value 222.

The progress procedure display region 230 includes a progress procedure display field 231, a parameter button 233, and the like. In the progress procedure display field 231, an examination program of the progress procedure set for the self-optometry being executed is displayed. In the example illustrated in FIG. 5, among the entire progress procedure (that is, a plurality of examinations included in the progress procedure), a procedure being performed at that time (an examination item being executed during the examination) is identifiably displayed. In the example, as the identification display, the executed examination item is displayed on the progress procedure by an arrow 232 and a thick frame. The progress procedure display field 231 displays a part (in FIG. 5, only the progress procedure for the left eye) of an example of the progress procedure of the self-optometry used in the subjective optometry device 100 according to the present example. The parameter button 233 is a button for displaying the parameter screen 220. When the amount of correction of the subject eye exceeds the allowable value, an alert mark 234 is displayed on the parameter button 233.

For example, when it is detected that the current amount of astigmatic axis correction is not within the allowable value, the controller 60 displays an alert on the self-optometry assistance screen 200. The alert of the self-optometry assistance screen 200 may be configured to warn the examiner that the current amount of astigmatic axis correction exceeds the allowable value.

For example, the controller 60 may invert the color of the procedure 231 in the progress procedure display field 231. For example, the controller 60 may display the alert mark 234 on the parameter button 233. For example, the alert mark 234 may be a badge indicating that there is a notification. Of course, the mark may be different from the badge as long as the presence or absence of the notification can be checked. For example, by checking the change in the color of the progress procedure display field 231 and the display of the alert mark 234, the examiner can recognize that a problem may have occurred in the progress of the cross cylinder test.

Further, when the examiner presses the parameter button 233, the controller 60 may display the parameter screen 220 based on the input signal of the parameter button 233. For example, the value 221 of the objective eye refractive power of the subject eye and the current correction value 222 of the subject eye are displayed on the parameter screen 220. For example, in a case where the amount of correction of the subject eye exceeds the allowable value, the value 221 may be highlighted (for example, bold, underline, changed in text color, or the like). For example, the examiner checks the current correction value 222 (in the present embodiment, the amount of astigmatic axis correction) on the parameter screen 220 and checks the examination status of the subject. For example, the examiner may continue with the self-optometry as it is, may interrupt the self-optometry and perform a manual examination, or may perform the cross cylinder test (A) again from the beginning as necessary.

In the above description, a case where an alert (as an example, the alert mark 234) is displayed on the self-optometry assistance screen 200 in <Output Alert> after executing the control from <Align Subject Eye> to <Compare Amount of Astigmatic Axis Correction with Allowable Value> is described as an example, but the present invention is not limited thereto. For example, based on the result of the comparison process as to whether the current amount of astigmatic axis correction of the subject eye E falls within the allowable value, the cross cylinder test (A) currently being performed may be stopped, and the self-optometry may be forcibly terminated without executing the next cross cylinder test (C) or subsequent tests. Further, for example, based on the result of the comparison process as to whether the current amount of astigmatic axis correction of the subject eye E falls within the allowable value, the cross cylinder test (A) currently being performed may be omitted, and the next cross cylinder test (C) may be progressed. In other words, the examination item may be automatically shifted such that the cross cylinder test (A) currently being performed is interrupted, the acquisition of the examination result of the amount of astigmatic axis correction in the cross cylinder test (A) is skipped, and the next cross cylinder test (C) is to be executed.

### <Interrupt Cross Cylinder Test (A)>

First, a case where the cross cylinder test (A) is stopped and the self-optometry is forcibly terminated will be described as an example. Here, the control from <Align Subject Eye> to <Compare Amount of Astigmatic Axis Correction with Allowable Value> is the same, and thus the description thereof will be omitted.

For example, the controller 60 detects whether the amount of correction which is sequentially changed during the cross cylinder test of the subject eye exceeds the allowable value which is based on the objective eye refractive power. In addition, for example, when it is detected that the current amount of correction of the subject eye is not within the allowable value, the controller 60 stops the cross cylinder test (A) currently being performed. Here, if the current amount of astigmatic axis correction of the subject eye is not within the range from 15 degrees to 45 degrees, which is the allowable value of the cross cylinder test (A), the cross cylinder test (A) is automatically ended. For example, the controller 60 may store the examination result of the cross cylinder test (A) for the subject eye E in the memory 70 as not applicable (for example, N/A). Further, for example, the controller 60 may store the examination result of the cross cylinder test (A) for the subject eye E in the memory 70 as the current correction value of the subject eye (in other words, the correction value immediately before the stop).

For example, when the examination result of the cross cylinder test (A) for the subject eye E is acquired, the controller 60 cancels the execution of the cross cylinder test (C), the red-green test, and the VA (visual acuity) test scheduled after the cross cylinder test (A), and forcibly terminates the self-optometry. For example, the examination result for each of the canceled examination items may be stored in the memory 70 as not applicable.

For example, as described above, the subject may continue to give vague answers in the cross cylinder test (A). In particular, when the cylindrical power of the subject eye is weak, the difference between the two examination visual targets is small, which tends to lead to a series of vague answers, making it difficult to obtain reliable examination results. Therefore, by comparing the current correction value of the subject eye and the allowable value and stopping the examination of a predetermined examination item (here, the cross cylinder test (A)) according to the situation, the burden on the subject can be reduced. In addition, by not executing the examination item after the predetermined examination item, the possibility of obtaining unreliable examination results for those examination items can be reduced.

For example, in the self-optometry of the subject eye E, when the cross cylinder test (A) for the subject eye E is stopped and the execution of the subsequent tests is stopped, an alert may be displayed on the self-optometry assistance screen 200. As an example, at least one of a message to inform the examiner that the self-optometry is forcibly terminated, the alert mark 234, and the like may be displayed. Further, for example, a progress result display field 241 to be described later may be displayed on the self-optometry assistance screen 200 to indicate which one of the plurality of examination items has been executed.

### <Omit Cross Cylinder Test (A) and Transition to Cross Cylinder Test (C)>

Next, a case where the cross cylinder test (A) is omitted and the process proceeds to the cross cylinder test (C) will be described as an example. Here, the control from <Align Subject Eye> to <Compare Amount of Astigmatic Axis Correction with Allowable Value> is the same, and thus the description thereof will also be omitted.

For example, the controller 60 detects whether the amount of correction which is sequentially changed during the cross cylinder test of the subject eye exceeds the allowable value which is based on the objective eye refractive power. In addition, for example, when it is detected that the current amount of astigmatic axis correction of the subject eye is not within the allowable value, the controller 60 stops and automatically ends the cross cylinder test (A) currently being performed. For example, the controller 60 may store the examination result of the cross cylinder test (A) for the subject eye E in the memory 70 as not applicable. For example, the controller 60 may store the examination result of the cross cylinder test (A) for the subject eye E in the memory 70 as the current correction value of the subject eye.

For example, when the examination result of the cross cylinder test (A) for the subject eye E is acquired, the controller 60 shifts the examination item to the cross cylinder test (C) scheduled after the cross cylinder test (A). For example, the controller 60 drives the left drive unit 53L to rotate the autocross cylinder lens of the left examination window 43L to an axial angle for adjusting the cylindrical power. In addition, for example, the controller 60 generates a voice guide from the speaker 3, asking the subject to indicate a direction of the point group visual target that can be clearly visually recognized. For example, the subject operates the subject controller 20 to tilt the answer lever 21 in the direction of the clearly visible point group visual target of the two point group visual targets.

For example, the controller 60 may also detect whether the amount of correction of the subject eye exceeds the allowable value which is based on the objective eye refractive power by each comparison process in the cross cylinder test (C). For example, in a case where it is detected that the current amount of cylindrical correction of the subject eye is not within the allowable value, the controller 60 may similarly omit the cross cylinder test (C) currently being performed, and may shift to the red-green test which is the next examination item. For example, the controller 60 may allow a series of examinations to progress to the end without interruption so as to execute all examination items scheduled in the self-optometry of the subject eye E.

For example, when it is difficult to obtain a reliable examination result for each examination item for the subject eye, the examination is likely to be prolonged. In the cross cylinder test (A) of the subject eye, such a possibility increases particularly when the cylindrical power is weak. Therefore, by comparing the current correction value of the subject eye and the allowable value and omitting the examination of a predetermined examination item (here, the cross cylinder test (A)) according to the situation and shifting to the next text, the self-optometry can be smoothly performed to the end.

When the cross cylinder test (A) for the subject eye E is omitted and the process proceeds to the cross cylinder test (C), an alert may be displayed on the self-optometry assistance screen 200 so that the examiner can grasp the omission of the cross cylinder test (A).

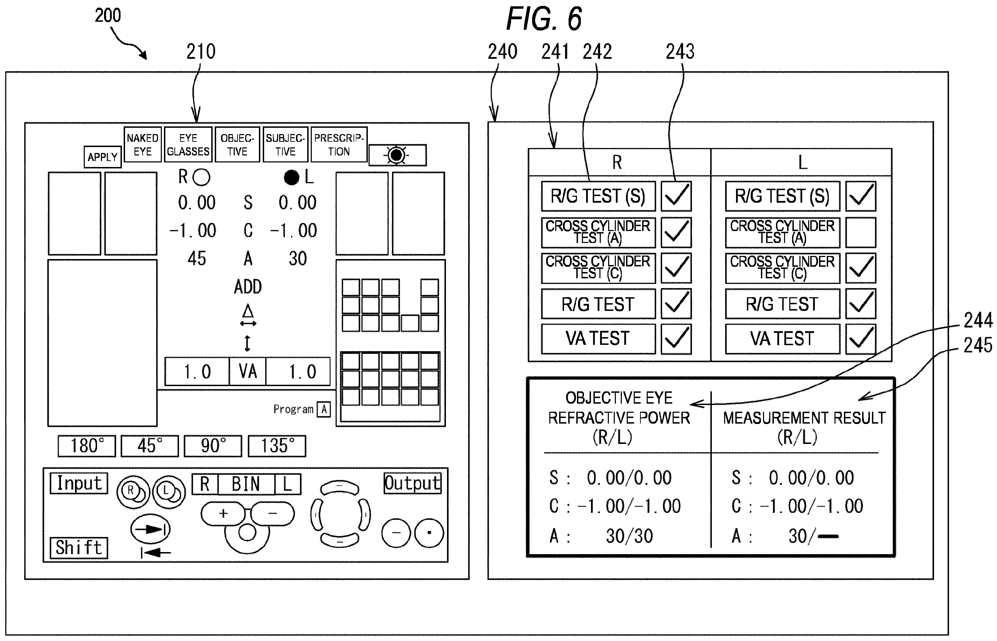

FIG. 6 is an example of the self-optometry assistance screen displayed on the monitor 12 of the examiner controller 10. For example, the self-optometry assistance screen 200 includes the operation image region 210, an examination result display region 240, and the like. For example, when the self-optometry for the subject eye E proceeds to the end, the examination result display region 240 may be displayed instead of the progress procedure display region 230. For example, the progress result display field 241, an objective eye refractive power value 244, a measurement value 245 of the optical characteristic (eye refractive power) of the subject eye, and the like are displayed in the examination result display region 240.

The progress result display field 241 is a display field indicating an execution status of each examination item of the self-optometry for the subject eye. For example, the progress result display field 241 includes an examination item 242, an examination completion confirmation field 243, and the like. For example, the examination item 242 is a list of examination items set in the self-optometry for the subject eye. As an example, the red-green test (S), the cross cylinder test (A), the cross cylinder test (C), the red-green test, and the VA (visual acuity) test are listed according to the self-optometry examination program. For example, the examination completion confirmation field 243 is a list of whether an examination item set in the self-optometry of the subject eye has been executed. For example, the examination completion confirmation field 243 is represented by a check box. For example, if the examination item is executed, the check box is checked, and if the examination item is not executed, the check box is unchecked. In the example, since the cross cylinder test (A) is omitted in the self-optometry for the subject eye, only the check box of the examination completion confirmation field 243 corresponding to the cross cylinder test (A) in the examination item 242 is left blank. For example, the examination completion confirmation field 243 may be different from the check box as long as the examiner can be notified that an examination item has been omitted.

For example, the objective eye refractive power value 244 and the measurement value 245 of the optical characteristic of the subject eye are displayed in a comparable manner. As an example, the objective eye refractive power value 244 and the measurement value 245 of the optical characteristic of the subject eye are displayed in parallel. For example, in the present example, since the cross cylinder test (A) of the subject eye is omitted, when the examination result of the cross cylinder test (A) is stored in the memory 70 as not applicable, the measurement value 245 is blank or displayed as an error.

For example, when the examination result of the cross cylinder test (A) is stored in the memory 70 as the correction value immediately before the examination is stopped, the correction value may be displayed as the measurement value 245. At this time, the controller 60 may blink or change the color of the corresponding measurement value 245 in order to allow the examiner to distinguish that the correction value is displayed as the measurement value 245. Of course, the controller 60 may display a message or the like for notifying the examiner that the correction value is displayed. For example, this allows the examiner to easily grasp that the reliability of the predetermined measurement value may be low.

For example, the examiner checks the execution status and the measurement value of each examination item using the progress result display field 241 and the measurement value 245 of the optical characteristic of the subject eye in the examination result display region 240. For example, this allows the examiner to grasp that a predetermined examination item has been omitted, and to take an appropriate action such as performing a manual examination as necessary.

As described above, for example, the subjective optometry device of the present example includes: a first acquisition unit that acquires at least one of previous eyeglass data obtained by measuring optical characteristics of glasses worn by the subject, objective measurement data obtained by objectively measuring optical characteristics of the subject eye, and previous subjective measurement data obtained by subjectively measuring optical characteristics of the subject eye in the past; a second acquisition unit that acquires a correction value by which the subject eye is corrected by the correction unit during the progress of the optometry; a comparison processing unit that compares an allowable value based on at least one of the previous eyeglass data, the objective measurement data, and the previous subjective measurement data which are acquired by the first acquisition unit with the correction value acquired by the second acquisition unit; and an output unit that outputs an alert based on a comparison result of the comparison processing unit. For example, in a case where the correction value by which the subject eye is corrected by the correction unit greatly deviates from the value of the previous eyeglass data, the objective measurement data, or the previous subjective measurement data, there is a possibility that the examination does not progress correctly. However, in a self-optometry in which a plurality of examination items for a subject eye are automatically progressed, it is difficult for the examiner to check whether the examination is correctly progressed since the examiner is often not near the subject. Therefore, for example, by outputting an alert in a case where the correction value of the subject eye exceeds the allowable value based on the previous eyeglass data, the objective measurement data, or the previous subjective measurement data, the examiner can easily check the examination status, and the examination can be performed smoothly.

In addition, for example, in the subjective optometry device according to the present embodiment, the second acquisition unit is configured to acquire the correction value at a predetermined timing during a predetermined examination item, the comparison processing unit is configured to compare the allowable value with the correction value acquired at the predetermined timing, and the output unit is configured to output the alert during the predetermined examination item. For example, in the self-optometry in which a plurality of examination items for a subject eye are automatically progressed, the examiner cannot check the examination result until all the examination items are completed. Therefore, for example, by providing the allowable value based on the previous eyeglass data, the objective measurement data, or the previous subjective measurement data which are acquired by the first acquisition unit and outputting an alert at a timing when the correction value during the examination acquired by the second acquisition unit exceeds the allowable value, the examiner can easily determine whether the examination is in progress without any problem.

In addition, for example, in the subjective optometry device according to the present embodiment, a predetermined timing may be a timing each time the control unit controls the correction unit to change the optical characteristic of the visual target light flux. For example, this allows the examiner to easily determine the next operation, such as continuing with the examination as it is or interrupting the examination or performing the examination again.

In addition, for example, in the subjective optometry device according to the present embodiment, at least one of the previous eyeglass data, the objective measurement data, and the previous subjective measurement data which are acquired by the first acquisition unit includes astigmatic axis data, the second acquisition unit is configured to acquire at least an astigmatic axis angle as the correction value, and the comparison processing unit is configured to compare the allowable value based on the astigmatic axis data with the astigmatic axis angle. For example, in the examination of the astigmatic axis angle, the subject is required to select one of the examination visual targets having a different appearance. However, in some cases, the selection is difficult, and there is a possibility that the examination result deviates from the previous eyeglass data, the objective measurement data, or the previous subjective measurement data. In addition, for example, when the examination result of the astigmatic axis angle is significantly deviated, the progress of the subsequent examination may be affected. As an example, there is a possibility that an appropriate cylindrical correction power cannot be determined in a cylindrical power test, a possibility that a predetermined visual acuity cannot be obtained in a visual acuity test, and the like. Therefore, the examiner can easily check the examination status by comparing the allowable value based on the astigmatic axis data acquired by the first acquisition unit with the astigmatic axis angle acquired by the second acquisition unit.

In addition, for example, in the subjective optometry device according to the present embodiment, the control unit is configured to, based on a comparison result of the comparison processing unit, omit a predetermined examination item among the plurality of examination items in the optometry and progress a next examination item. For example, when a plurality of examination items for the subject eye are automatically progressed, a reliable examination result cannot be easily obtained in a predetermined examination item depending on an optical characteristic of the subject eye, and the examination may continue for a long time. Therefore, for example, by omitting such a predetermined examination item and shifting to the next examination item, the self-optometry can be smoothly progressed to the end.

In addition, for example, in the subjective optometry device according to the present embodiment, the control unit is configured to, based on a comparison result of the comparison processing unit, forcibly terminate the optometry without executing a predetermined examination item among the plurality of examination items in the optometry. For example, in the self-optometry of the subject eye, in a case where a reliable examination result cannot be easily obtained and there is an examination item continuing for a long time as described above, the burden on the subject can be reduced by stopping the examination of the predetermined examination item. Further, for example, since the examination result of the predetermined examination item affects the examination result of the subsequent examination items, it is possible to reduce the possibility of obtaining an examination result with low reliability by not performing the subsequent examination items.

In addition, for example, in the subjective optometry device according to the present embodiment, the predetermined examination item is an examination item for acquiring the astigmatic axis angle as the correction value of the subject eye. For example, in the examination of the astigmatic axis angle, a subject is required to compare appearances of two examination visual targets and select one of the examination visual targets. For example, in a case where the cylindrical power of the subject eye is weak, the difference in the appearance of the examination visual targets is particularly small, increasing the chances of confusion in the selection. Therefore, it is difficult to obtain a highly reliable examination result, and the examination result may not be determined indefinitely. However, as in the present embodiment, by omitting or forcibly terminating the examination item for acquiring the astigmatic axis angle based on the comparison result of the allowable value with the correction value, the self-optometry can be smoothly progressed.

### <Modifications>

In the subjective optometry device according to the present embodiment, the case where an alert is output during the progress of the examination of a predetermined examination item in the self-optometry for the subject eye has been described as an example, but the present disclosure is not limited thereto. For example, an alert may be output when the examination of each examination item is completed (for example, when the examination result is confirmed). In addition, for example, an alert may be output when all of the plurality of examination items in the self-optometry are completed (that is, all of the examination results are acquired). The subjective optometry device according to the present embodiment may be configured such that an alert is output at at least one timing of during the examination of a predetermined examination item, a time point when the examination of each examination item is completed, and a time point when all the examination items are completed.

For example, as described above, in the subjective optometry device according to the present embodiment, the output unit for outputting the alert based on a comparison result between the correction value of the subject eye and the allowable value may output the alert when the plurality of examination items are completed. For example, by outputting an alert at a timing when the plurality of examination items are completed, the examiner can easily determine whether there is a problem in the examination result.

In the present embodiment, a configuration in which the respective allowable values are uniformly set regardless of the values of the spherical power, the cylindrical power, and the astigmatic axis angle in the objective eye refractive power of the subject eye has been described as an example, but the present disclosure is not limited thereto. For example, a different allowable value may be set for each spherical power in the objective eye refractive power of the subject eye. In addition, for example, a different allowable value may be set for each cylindrical power in the objective eye refractive power of the subject eye. In addition, for example, a different allowable value may be set for each astigmatic axis angle in the objective eye refractive power of the subject eye.

For example, as described above, in the subjective optometry device according to the present embodiment, the allowable value based on at least one of the previous eyeglass data, the objective measurement data, and the previous subjective measurement data which are acquired by the first acquisition unit is set for each optical characteristic of the previous eyeglass data, the objective measurement data, or the previous subjective measurement data which are acquired by the first acquisition unit. For example, the likelihood of deviation in the examination result varies depending on the magnitude of the absolute value of the optical characteristic based on the previous eyeglass data, the objective measurement data, or the previous subjective measurement data. Therefore, for example, when the allowable value is uniformly set regardless of the optical characteristics of the previous eyeglass data, the objective measurement data, or the previous subjective measurement data, there is a possibility that the alert is frequently output due to a small allowable value, or there is a possibility that the alert cannot be output even though the deviation occurs due to a large allowable value. Therefore, for example, as in the present embodiment, since a different allowable value is set for each optical characteristic of the previous eyeglass data, the objective measurement data, or the previous subjective measurement data, the alert is appropriately output, so that the examiner can easily determine whether there is a problem in the examination result.

In the subjective optometry device according to the present embodiment, the configuration in which the allowable value of the amount of astigmatic axis correction of the subject eye is set based on the astigmatic axis angle in the objective eye refractive power of the subject eye has been described as an example, but the present disclosure is not limited thereto. For example, the allowable value of the amount of astigmatic axis correction may be determined based on the cylindrical power in the objective eye refractive power of the subject eye.

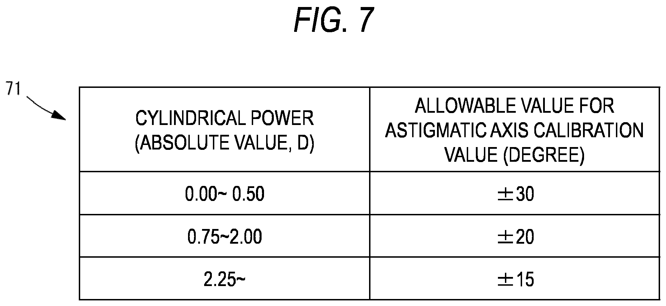

FIG. 7 is a diagram illustrating a correspondence relationship between the cylindrical power in the objective eye refractive power of the subject eye and the allowable value of the amount of astigmatic axis correction for correcting the subject eye. For example, a lookup table 71 in which the cylindrical power in the objective eye refractive power of the subject eye and the allowable value of the amount of astigmatic axis correction for correcting the subject eye are associated with each other may be created by performing experiments or simulations in advance and be stored in the memory 70. For example, in the lookup table 71, the smaller the absolute value of the cylindrical power in the objective eye refractive power of the subject eye, the larger the allowable value is set, and the larger the absolute value of the cylindrical power in the objective eye refractive power of the subject eye, the smaller the allowable value is set. As an example, when the cylindrical power in the objective eye refractive power of the subject eye is -1.00 D and the astigmatic axis angle is 20 degrees, the allowable value of the amount of astigmatic axis correction of the subject eye is set to a range from 0 degrees to 40 degrees with reference to the lookup table 71. The controller 60 may detect whether the amount of astigmatic axis correction of the subject eye falls within the range from 0 degrees to 40 degrees by a comparison process. Of course, the lookup table 71 described above is an example, and the range of the cylindrical power in the objective eye refractive power of the subject eye and the allowable value of the amount of astigmatic axis correction may be freely added or changed.

For example, as described above, in the subjective optometry device according to the present embodiment, at least one of the previous eyeglass data, the objective measurement data, and the previous subjective measurement data which are acquired by the first acquisition unit includes the cylindrical data, and the allowable value is set more strictly as the refractive power of the cylindrical data becomes higher. For example, the smaller the absolute value of the cylindrical power of the subject eye (that is, the lower the refractive power), the less likely a difference is to be felt in the appearance of the two examination visual targets in the astigmatic axis examination, and thus the astigmatic axis angle is less likely to be affected by the axis deviation in the examination result. On the other hand, the larger the absolute value of the cylindrical power of the subject eye (that is, the higher the refractive power), the more likely a difference is to be felt in the appearance of the two examination visual targets in the astigmatic axis examination, and thus the astigmatic axis angle is more likely to be affected by the axis deviation in the examination result. Therefore, as in the present embodiment, by setting the allowable value of the astigmatic axis data more strictly as the refractive power of the cylindrical data becomes higher, the examiner can easily determine whether there is a problem in the progress of the astigmatic axis examination, and can acquire correct examination result.

In the subjective optometry device according to the present embodiment, a configuration in which the color of the procedure 231 of the progress procedure display field 231 is reversed and the alert mark 234 is displayed on the parameter button 233 on the self-optometry assistance screen (see FIG. 5) has been described as an example, but the present disclosure is not limited thereto. For example, a configuration may be adopted in which either the color inversion of the procedure 231 or the display of the alert mark 234 is executed. Instead of the color inversion of the procedure 231 or the display of the alert mark 234, or together with the color inversion of the procedure 231 or the display of the alert mark 234, an alert may be output to the examiner by issuing a notification by generating sound or oscillation, or by blinking a pilot lamp or the like.

The alert of the present embodiment may be different from the above. For example, at least one of the arrow 232, the progress procedure display region 220, and the like displayed to identify the executed examination item may be configured to be reversed in color, displayed in a blinking manner, or the like. In addition, for example, a configuration in which an alert mark is displayed on the examination item in progress in the procedure 231, a configuration in which the current correction value is displayed near the examination item in progress in the procedure 231, a configuration in which at least one of color inversion and blinking display of the examination item in progress in the procedure 231 is executed, or the like may be adopted.

In the present embodiment, the case where the allowable value based on the objective eye refractive power (objective measurement data) of the subject eye is set has been described as an example. The present disclosure is not limited thereto. In the present embodiment, it is also possible to set an allowable value based on lens information of the previous eyeglass (previous eyeglass data) of the subject. In addition, it is also possible to set an allowable value based on a result of a subjective measurement of the subject eye acquired in the past (previous subjective measurement data).

For example, in a case where the allowable value based on the previous subjective measurement data acquired in the past is set, an allowable value based on the result of the subjective measurement acquired at a date and time different from the current time point of examining the subject eye may be set. In addition, for example, in a case where the allowable value based on the previous subjective measurement data acquired in the past is set, the allowable value may be set based on the result of the subjective measurement acquired in an examination item executed before a predetermined examination item currently being performed among a plurality of examination items for examining the subject eye. As an example, an allowable value in a second red-green test may be set based on the result of a first red-green test. As an example, an allowable value in the visual acuity test may be set based on the results of the red-green test and the cross cylinder test. That is, the allowable value may be appropriately changed according to the examination result obtained by sequentially progressing the plurality of examination items for the subject eye.

In the present embodiment, a configuration in which the controller 60 included in the subjective optometry device 100 acquires the objective eye refractive power (for example, objective measurement data) of the subject eye and the correction value of the subject eye in the subjective measurement, compares the allowable value based on the objective eye refractive power with the correction value, and outputs an alert has been described as an example, but the present disclosure is not limited thereto. For example, a control unit of a medium (for example, a computer, a terminal device, or the like) communicably connected to the main body of the subjective optometry device 100 may acquire the objective eye refractive power and the correction value of the subject eye, compare the allowable value based on the objective eye refractive power with the correction value, and output the alert. In addition, for example, the controller 60 included in the subjective optometry device 100 may perform a process of comparing the allowable value based on the objective eye refractive power with the correction value, and the control unit of the medium communicably connected to the main body of the subjective optometry device 100 may output the alert. In this case, for example, the controller 60 of the subjective optometry device 100 transmits a signal to the medium communicably connected to the subjective optometry device based on a comparison result of the comparison processing unit. For example, the control unit of the medium communicably connected to the subjective optometry device may output an alert via the medium by receiving a signal from the controller 60.

In the subjective optometry device according to the present embodiment, the configuration in which a predetermined examination item is omitted or the self-optometry is forcibly terminated and an alert is further output in a case where the amount of correction for correcting the subject eye exceeds the allowable value based on the objective eye refractive power has been described as an example, but the present disclosure is not limited thereto. The subjective optometry device according to the present embodiment may have a configuration in which the alert is not necessarily output, as long as a predetermined examination item is omitted or the self-optometry is forcibly terminated in a case where the amount of correction of the subject eye exceeds the allowable value. In this case, for example, the examiner can grasp whether the examination of the predetermined examination item has progressed without any problem by comparing a value of the objective eye refractive power of the subject eye with a correction value immediately before the stop of the predetermined examination item of the subject eye.

For example, in this case, the subjective optometry device according to the present embodiment is for subjectively measuring an optical characteristic of a subject eye, the subjective optometry device including: a visual target presenting unit configured to present an examination visual target to a subject eye; a correction unit configured to change an optical characteristic of a visual target light flux emitted from the visual target presenting unit; a control unit configured to, based on an answer input by a subject, control at least one of the visual target presenting unit and the correction unit, and automatically progress an optometry including a plurality of examination items; a first acquisition unit configured to acquire at least one of previous eyeglass data obtained by measuring an optical characteristic of eyeglasses worn by a subject, objective measurement data obtained by objectively measuring an optical characteristic of the subject eye, and previous subjective measurement data acquired by subjectively measuring an optical characteristic of the subject eye previously; a second acquisition unit configured to acquire a correction value by which the subject eye is corrected by the correction unit during the optometry; and a comparison processing unit configured to compare an allowable value that is based on at least one of the previous eyeglass data, the objective measurement data, and the previous subjective measurement data which are acquired by the first acquisition unit with the correction value that is acquired by the second acquisition unit. The control unit may omit a predetermined examination item among a plurality of examination items in the optometry based on a comparison result of the comparison processing unit, and progress the next examination item.

In addition, for example, in this case, the subjective optometry device according to the present embodiment is for subjectively measuring an optical characteristic of a subject eye, the subjective optometry device including: a visual target presenting unit configured to present an examination visual target to a subject eye; a correction unit configured to change an optical characteristic of a visual target light flux emitted from the visual target presenting unit; a control unit configured to, based on an answer input by a subject, control at least one of the visual target presenting unit and the correction unit, and automatically progress an optometry including a plurality of examination items; a first acquisition unit configured to acquire at least one of previous eyeglass data obtained by measuring an optical characteristic of eyeglasses worn by a subject, objective measurement data obtained by objectively measuring an optical characteristic of the subject eye, and previous subjective measurement data acquired by subjectively measuring an optical characteristic of the subject eye previously; a second acquisition unit configured to acquire a correction value by which the subject eye is corrected by the correction unit during the optometry; and a comparison processing unit configured to compare an allowable value that is based on at least one of the previous eyeglass data, the objective measurement data, and the previous subjective measurement data which are acquired by the first acquisition unit with the correction value that is acquired by the second acquisition unit. The control unit may, based on a comparison result of the comparison processing unit, forcibly terminate the optometry without executing a predetermined examination item among the plurality of examination items in the optometry.

In the subjective optometry device according to the present embodiment, a configuration in which the measurement value 245 of the optical characteristic of the subject eye is blank or displayed as an error in a case where the amount of correction for correcting the subject eye exceeds the allowable value based on the objective eye refractive power has been described as an example, but the present disclosure is not limited thereto. In the subjective optometry device according to the present embodiment, in a case where the correction amount of the subject eye exceeds the allowable value, at least one of the optical characteristic of the eyeglass lens worn by the subject (that is, the previous eyeglass data), the value of the objective eye refractive power of the subject eye (that is, the objective measurement data), and the measurement result of the past subjective measurement of the subject (that is, the previous subjective measurement data) may be displayed as the measurement value 245 of the optical characteristic of the subject eye. For example, in this case, one or more of the previous eyeglass data, the objective measurement data, and the previous subjective measurement data may be set to be displayed in advance. In addition, for example, in this case, one or more of the previous eyeglass data, the objective measurement data, and the previous subjective measurement data may be freely selected and displayed.

For example, as described above, in the subjective optometry device according to the present embodiment, in a case where a predetermined examination item in the optometry is omitted or the optometry is forcibly terminated, the control unit acquires at least one of previous eyeglass data described later, objective measurement data described later, and previous subjective measurement data described later as an examination result of the predetermined examination item. For example, when the predetermined examination item is omitted, an examination result with high reliability cannot be acquired for the predetermined examination item. Further, for example, in a case where the optometry is forcibly terminated, a highly reliable examination result cannot be acquired for the predetermined examination item, and since the examination is not performed after the predetermined examination item, there is no examination result. Therefore, for example, by acquiring at least one of the previous eyeglass data, the objective measurement data, and the previous subjective measurement data of the subject eye as reference data of the examination result in the predetermined examination item, the examiner can easily grasp the optical characteristics of the subject eye.

### REFERENCE SIGNS LIST

1 housing
2 presentation window
3 speaker
10 examiner controller
20 subject controller
30 light projection optical system
40 eye refractive power measurement unit
43 examination window
60 controller
100 subjective optometry device

## Claims

1. A subjective optometry device for subjectively measuring an optical characteristic of a subject eye, the subjective optometry device comprising:
a visual target presenting unit configured to present an examination visual target to a subject eye;
a correction unit configured to change an optical characteristic of a visual target light flux emitted from the visual target presenting unit;
a controller configured to control at least one of the visual target presenting unit and the correction unit, based on an answer input by a subject, to automatically progress an optometry including a plurality of examination items;
a first acquisition unit configured to acquire at least one of previous eyeglass data obtained by measuring an optical characteristic of eyeglasses worn by a subject, objective measurement data obtained by objectively measuring an optical characteristic of the subject eye, and previous subjective measurement data acquired by subjectively measuring an optical characteristic of the subject eye previously;
a second acquisition unit configured to acquire a correction value by which the subject eye is corrected by the correction unit during the optometry;
a comparison processing unit configured to compare an allowable value with the correction value that is acquired by the second acquisition unit, the allowable value being based on at least one of the previous eyeglass data, the objective measurement data, and the previous subjective measurement data that are acquired by the first acquisition unit; and
an output unit configured to output an alert based on a comparison result of the comparison processing unit.

2. The subjective optometry device according to claim 1,
wherein the second acquisition unit is configured to acquire the correction value at a predetermined timing during a predetermined examination item,
wherein the comparison processing unit is configured to compare the allowable value with the correction value acquired at the predetermined timing, and
wherein the output unit is configured to output the alert during the predetermined examination item.

3. The subjective optometry device according to claim 2,
wherein the predetermined timing is a timing each time the controller controls the correction unit to change an optical characteristic of the visual target light flux.

4. The subjective optometry device according to any one of claims 1 to 3,
wherein in a case where the plurality of examination items are completed, the output unit outputs the alert.

5. The subjective optometry device according to any one of claims 1 to 4,
wherein the allowable value is set for each optical characteristic of the previous eyeglass data, the objective measurement data, or the previous subjective measurement data that are acquired by the first acquisition unit.

6. The subjective optometry device according to any one of claims 1 to 5,
wherein at least one of the previous eyeglass data, the objective measurement data, and the previous subjective measurement data that are acquired by the first acquisition unit includes astigmatic axis data,
wherein the second acquisition unit is configured to acquire at least an astigmatic axis angle as the correction value, and
wherein the comparison processing unit is configured to compare the allowable value based on the astigmatic axis data with the astigmatic axis angle.

7. The subjective optometry device according to claim 6,
wherein at least one of the previous eyeglass data, the objective measurement data, and the previous subjective measurement data that are acquired by the first acquisition unit includes cylindrical data, and
wherein the allowable value is set more strictly as refractive power of the cylindrical data increases.

8. The subjective optometry device according to any one of claims 1 to 7,
wherein the controller is configured to omit a predetermined examination item among the plurality of examination items in the optometry, based on a comparison result of the comparison processing unit, to progress a next examination item.

9. The subjective optometry device according to any one of claims 1 to 8,
wherein the controller is configured not to perform a predetermined examination item among the plurality of examination items in the optometry, based on a comparison result of the comparison processing unit, to forcibly terminate the optometry.

10. The subjective optometry device according to claim 8 or 9,
wherein the controller is configured to, in a case where the predetermined examination item in the optometry is omitted or the optometry is forcibly terminated, acquire at least one of previous eyeglass data, the objective measurement data, and the previous subjective measurement data as an examination result of the predetermined examination item.

11. The subjective optometry device according to any one of claims 8 to 10,
wherein the predetermined examination item is an examination item for acquiring the astigmatic axis angle as the correction value of the subject eye.

12. A subjective optometry program to be used in a subjective optometry device for subjectively measuring an optical characteristic of a subject eye,
the subjective optometry device including:
a visual target presenting unit configured to present an examination visual target to a subject eye;
a correction unit configured to change an optical characteristic of a visual target light flux emitted from the visual target presenting unit; and
a controller configured to control at least one of the visual target presenting unit and the correction unit, based on an answer input by a subject, to automatically progress an optometry including a plurality of examination items,
the subjective optometry program comprising instructions which, when executed by a processor of the subjective optometry device, cause the processor to execute:
a first acquisition step of acquiring at least one of previous eyeglass data obtained by measuring an optical characteristic of eyeglasses worn by a subject, objective measurement data obtained by objectively measuring an optical characteristic of the subject eye, and previous subjective measurement data acquired by subjectively measuring an optical characteristic of the subject eye previously;
a second acquisition step of acquiring a correction value by which the subject eye is corrected by the correction unit during the optometry;
a comparison processing step of comparing an allowable value with the correction value that is acquired in the second acquisition step, the allowable value being based on at least one of the previous eyeglass data, the objective measurement data, and the previous subjective measurement data that are acquired in the first acquisition step; and
an output step of outputting an alert based on a comparison result of the comparison processing step.

13. The subjective optometry program according to claim 12,
wherein the controller is configured to omit a predetermined examination item among the plurality of examination items in the optometry, based on a comparison result of the comparison processing step, to progress a next examination item.

14. The subjective optometry program according to claim 13,
wherein the controller is configured not to execute a predetermined examination item among the plurality of examination items in the optometry, based on a comparison result of the comparison processing step, to forcibly terminate the optometry.
